# FASCICULE DE BREVET EUROPEEN

(11) **EP 2 892 482 B1**
(45) Date de publication et mention de la délivrance du brevet: **08.11.2017**
(21) Numéro de dépôt: 13766611.1
(22) Date de dépôt: 03.09.2013
(51) Int. Cl.: A61F 13/06, A61F 13/02

(54) **NOUVELLE BANDE ELASTIQUE AUTOADHERENTE UTILISABLE NOTAMMENT POUR LE TRAITEMENT ET LA PREVENTION DES PATHOLOGIES D'ORIGINE VEINEUSE.**
NEUARTIGE SELBSTKLEBENDE ELASTISCHE BANDAGE, INSBESONDERE ZUR BEHANDLUNG UND PRÄVENTION VON VENENERKRANKUNGEN
NOVEL SELF-ADHESIVE ELASTIC BANDAGE THAT CAN BE USED, IN PARTICULAR, FOR THE TREATMENT AND PREVENTION OF DISEASES OF THE VEINS

(30) Priorité: 03.09.2012 FR 1258184
(43) Date de publication de la demande: 15.07.2015
(73) Titulaire: Urgo Recherche Innovation et Développement, 21300 Chenove (FR)
(72) Inventeur: PERNOT, Jean-Marc, Henri, Maurice, F-21000 Dijon (FR); LECOMTE, Serge, F-21000 Dijon (FR)
(74) Mandataire: Chantraine, Sylvie Hélène
(86) Numéro de dépôt international: PCT/FR2013/052023
(87) Numéro de publication internationale: WO 2014/033417

(56) Documents cités:
- EP-A1- 2 058 424
- US-A- 5 939 339
- US-A1- 2003 040 691
- US-A1- 2011 208 101

## Description

La présente invention est relative à une nouvelle bande de contention autoadhérente, notamment pour le traitement et la prévention des pathologies d'origine veineuse et des lymphoedèmes. Cette bande est fabriquée par l'assemblage de deux non tissés autoadhérents obtenus à partir de fibres courtes conjuguées qui ont été frisées. Les fibres sont de préférence en polyester. La bande peut en outre comprendre une couche supplémentaire.

L'utilisation de divers systèmes de contention est connue depuis longtemps pour traiter les pathologies d'origine veineuse, comme par exemple l'insuffisance veineuse, le traitement des varices et des ulcères de jambes ou encore pour prévenir la thrombose veineuse ou le traitement des lymphoedèmes. Ces systèmes sont constitués d'une ou de plusieurs bandes élastiques qui appliquent une pression sur le membre à traiter.

L'application d'une pression adéquate agit favorablement:
- d'une part, au niveau des vaisseaux en réduisant le calibre des veines ce qui conduit à l'accélération du flux sanguin et au rétablissement de la fonction valvulaire; et
- d'autre part, au niveau des tissus en favorisant une meilleure oxygénation et une résorption de l'oedème.

Dans le traitement des plaies chroniques et tout particulièrement dans celui des ulcères de jambes, l'utilisation d'un système de contention - qui permet de rétablir ou de favoriser une circulation veineuse normale - est le traitement de référence. C'est la seule thérapie qui ait prouvé son efficacité pour soigner et éviter la récidive de ce type de plaies.

Un système de contention efficace doit permettre de répondre à quatre objectifs principaux.

Premièrement, le système doit pouvoir être porté de façon continue jour et nuit durant un ou plusieurs jours (par exemple une semaine) selon la pathologie, sa gravité ou l'objectif thérapeutique (traitement ou prévention).

A cet effet, ce système doit donc permettre d'appliquer simultanément :
- d'une part, une pression relativement faible appelée «pression de repos», quand le muscle est relâché pour être confortable et en particulier supportable durant la nuit; et
- d'autre part, une pression relativement élevée appelée «pression de travail», quand le muscle est tendu ou lors des mouvements, et en particulier pendant la marche.

Deuxièmement, le différentiel de pression entre pression de repos et pression de travail doit être suffisant pour favoriser le reflux veineux.

Troisièmement, les valeurs de pression au repos, de pression de travail et de différentiel de pression doivent être stables dans le temps.

Quatrièmement, le système doit être facile et rapide à poser, et ceci en toute sécurité, afin d'éviter les risques de garrot si la pression appliquée est trop élevée, ou d'inefficacité si la pression ou le différentiel de pression est trop faible.

Pour parvenir à ces objectifs, on a développé des bandes élastiques tricotées ou tissées appelées bandes de contention.

Lors de son application autour d'un membre comme la jambe, la bande est étirée; suivant le degré d'allongement, elle applique sur la jambe une pression plus ou moins élevée. Cette pression, qui est la pression de traitement, dépend principalement de deux facteurs, l'allongement de la bande à la pose et la circonférence du membre sur lequel cette dernière est appliquée.

Les bandes élastiques de contention sont donc enroulées à un allongement donné autour de la jambe. Lors de l'enroulement de la bande, on procède à un recouvrement plus ou moins total de celle-ci sur elle-même. Très souvent, ce recouvrement est de 50 % dans le sens de pose transversal de la bande.

On considère généralement qu'un différentiel de pression à 24 heures compris entre 15 et 25 mm de mercure est nécessaire pour rétablir un flux veineux correct. Toutefois selon la pathologie, qu'il s'agisse d'un traitement sur des jambes sans ulcère grave, d'un traitement difficile sur des jambes abimées par un oedème, ou d'un traitement d'un ulcère mixte artériel et veineux, cette plage de valeurs peut s'étendre de 10 à 35 mm de mercure voire même de 10 à 40 mm de mercure.

Pour les ulcères, on vise généralement une pression de travail appliquée à 24 heures comprise entre 20 et 60 mm de mercure selon les pathologies.

Dans le cas du traitement des lymphoedèmes, l'élément important est davantage la pression appliquée que le différentiel de pression, et l'on vise pour cette pathologie, par exemple pour le lymphoedème de la jambe, une pression de travail à 24 heures supérieure à 60 mm de mercure, de préférence comprise entre 65 et 100 mm de mercure.

Les fabricants fournissent généralement des tableaux et des gammes de produits qui, pour un diamètre de membre donné, permettent de déterminer la pression à appliquer et de choisir le système adéquat.

Les bandes de contention utilisées sont classées par les spécialistes de la contention en deux grandes catégories selon la mesure de leur allongement.

La classification est basée sur la mesure de l'allongement telle que définie dans la norme EN 14704-1 quand la bande est soumise à une force de traction maximale de 6 N/cm.

Les conditions de réalisation de la mesure sont les suivantes.

Une éprouvette du matériau à tester de 50 mm de largueur et de 250 à 300 mm de longueur est découpée et positionnée sans précontrainte dans les mors d'un dynamomètre électronique (par exemple un dynamomètre de marque MTS) de sorte à avoir une largeur de 50 mm et une longueur utile de référence de 200 mm. Le dynamomètre étire l'éprouvette à une vitesse de 100 mm/min jusqu'à une force maximale de 6 N/cm puis la traverse revient à sa position initiale à la même vitesse de retour de 100 mm/min. Ce cycle est effectué 5 fois et l'allongement obtenu au cinquième cycle, exprimé en pourcentage, est directement calculé par l'appareillage. L'opération est répétée sur 5 éprouvettes et on prend la valeur moyenne.

### 1) Les bandes dites à allongement court

Sur la base de ce test pris comme référence, on considère qu'une bande de contention est une bande à «allongement court» si son allongement est inférieur ou égal à 100%.

Ces bandes exercent une pression au repos basse et une pression de travail élevée. Elles ont donc un grand différentiel de pression en particulier lors des mouvements, par exemple pendant la marche.

Les bandes à allongement court possèdent toutefois de nombreux inconvénients.

Tout d'abord, elles sont difficiles à poser car de faibles variations d'allongement à la pose génèrent de fortes augmentations ou diminutions de la pression atteinte et du différentiel de pression obtenu. Il existe donc un risque de garrot si la pression appliquée est trop élevée, ou un risque d'inefficacité si elle est insuffisante.

On constate aussi une diminution significative au cours du temps de la pression appliquée et du différentiel de pression entre pression de travail et pression de repos, et souvent un glissement de la bande de contention.

Il en résulte la nécessité de procéder à des changements fréquents de ces bandes et une augmentation corrélative du coût du traitement.

Une bande à allongement court est par exemple commercialisée par la société ACTIVA sous la dénomination Actico®. Cette bande est enroulée sur une bande de ouate préalablement enroulée sur la jambe. La ouate est destinée à répartir les pressions à la surface du membre, et/ou à protéger par son épaisseur les saillies osseuses, et à absorber les éventuels exsudats si la bande est posée sur une plaie ouverte, par exemple dans le cas des ulcères de jambes.

### 2) Les bandes à allongement long

Sur la base du test précédent pris comme référence, on considère qu'une bande est une bande à «allongement long» si son allongement est supérieur à 100%.

Ces bandes sont plus faciles à poser car elles présentent une plus grande extensibilité. De ce fait, des variations d'allongement à la pose ne génèrent pas de variations importantes de la pression appliquée. Le risque de garrot est faible.

En revanche, ces bandes conduisent à de faibles variations de pression entre repos et travail, et à une faible variation de pression au cours des mouvements, par exemple lors de la marche. Elles s'avèrent moins efficaces que les bandes à allongement court.

Elles présentent aussi un certain inconfort en position de repos si l'on souhaite imposer une pression élevée, d'où la nécessité de les retirer la nuit en raison de la gêne occasionnée.

Des bandes à allongement long sont par exemple commercialisées par les sociétés THUASNE et SMITH ET NEPHEW respectivement sous les dénominations Biflex® et Proguide®.

Pour essayer de remédier à ces inconvénients, des systèmes multicouches ont été développés.

Tous les systèmes de contention aujourd'hui utilisés sont constitués soit d'une bande unique choisie parmi ces deux catégories, soit de l'association de plusieurs bandes choisies parmi ces deux catégories, si nécessaire en association avec une première couche de ouate en contact avec la peau.

Parmi les systèmes qui utilisent plusieurs bandes on connaît ainsi depuis les années 1980 des systèmes de contention efficaces qui sont constitués de quatre bandes. De tels systèmes sont par exemple commercialisés par les sociétés SMITH AND NEPHEW et URGO Limited respectivement sous les dénominations Profore® et K4®. Ces systèmes ont pour principal inconvénient qu'ils sont très longs à poser.

Pour optimiser ce système « quatre couches » et les systèmes qui utilisent une seule bande et les rendre plus performants par rapport à l'utilisation d'une bande unique ou de l'association de quatre couches - soit en termes de facilité et de rapidité de pose, soit en termes d'efficacité thérapeutique - des systèmes de contention constitués de deux bandes élastiques ont été récemment commercialisés par exemple par la société Laboratoires URGO sous les dénominations K2® et K2 Lite®. La première bande (commercialisée sous la dénomination Ktech®) est une bande à allongement court constituée d'une couche d'ouate venant au contact de la peau et aiguilletée à un tricot élastique. La seconde bande (commercialisée sous la dénomination KPress®) est élastique et autoadhérente. C'est une bande à allongement long qui sert à maintenir la première bande en place et à appliquer la pression complémentaire par rapport à la première bande pour obtenir la pression recherchée. Cette association permet une pose plus rapide et l'obtention d'une pression de travail et d'un différentiel de pression optimisés ainsi que leur bonne conservation au cours du temps. Ce système nécessite de poser deux bandes, ce qui prend toujours du temps et donne au final un système épais et parfois peu confortable ou conformable.

Un autre inconvénient de tous ces systèmes est que l'autoadhérence des bandes est obtenue à l'aide d'adhésif ou de latex ce qui complique leur mise au point et peut - en particulier dans le cas du latex de caoutchouc naturel - entraîner des risques d'allergie au contact de la peau.

Au final, malgré la réalisation de ces systèmes bicouches pour traiter les pathologies d'origine veineuse ou les lymphoedèmes, la mise au point d'un produit optimal n'a toujours pas été réalisée.

Afin d'améliorer l'acceptabilité par les patients et le personnel soignant, la précision et la rapidité de pose, ainsi que l'efficacité thérapeutique, il apparaît donc souhaitable de disposer d'un système de contention qui soit constitué d'une seule bande. Une telle bande devrait combiner les avantages des bandes à allongement court, des bandes à allongement long, ou de leur association tout en s'affranchissant de leurs défauts, à savoir :
- être facile et rapide à poser,
- être la moins épaisse possible pour améliorer son confort et sa conformabilité,
- ne pas utiliser de latex ou d'adhésif susceptible de venir au contact de la peau,
- appliquer et conserver au cours du temps la pression et le différentiel de pression recherchés, et
- être facile à fabriquer.

La réalisation d'une bande unique présentant à la fois les avantages des bandes à allongement court et les avantages des bandes à allongement long tout en s'affranchissant de leurs défauts n'a jamais été décrite.

C'est l'objet de la présente invention que de fournir un système de contention efficace ne comprenant qu'une seule bande et qui remplit ce cahier des charges très complexe. Cette bande est une bande élastique autoadhérente qui est obtenu grâce à l'assemblage d'au moins deux non tissés spécifiques qui ont été obtenus à partir de fibres conjuguées courtes qui ont été frisées.

Des non tissés extensibles sont décrits dans la demande de brevet WO 2008/015972, pour leur utilisation comme bande de maintien ou de bandage. Les bandes décrites dans ce document sont néanmoins trop fragiles pour que leur application comme bande de contention puisse être envisagée. Une bande de contention doit en effet être suffisamment solide pour résister à des déformations, des frottements et des tensions répétées pendant plusieurs jours, principalement au niveau du talon et de la malléole. Les bandes de maintien constituées des non tissés décrites dans la demande WO 2008/015972 sont trop facilement perforables et ne peuvent être utilisées dans le domaine de la thérapie compressive comme bandes de contention.

Ces non tissés ne correspondent ni à des bandes à allongement long ni à des bandes à allongement court. En effet, si on tente de mesurer leur allongement selon la norme EN 14704-1 qui caractérise les bandes de contention, ils cassent dès le premier cycle avant d'avoir atteint la force de traction maximale de 6 N/cm.

De façon tout à fait surprenante, le déposant a obtenu une bande de contention efficace par l'assemblage d'au moins deux de ces non tissés. Cette bande présente des propriétés remarquables qui la rendent particulièrement adaptée à une utilisation en thérapie compressive.

On obtient une seule bande dont les propriétés - en termes de maintien de la pression appliquée et du différentiel de pression dans le temps - sont supérieures à celles des systèmes de contention aujourd'hui utilisés, et en particulier les systèmes bicouches les plus performants. La bande de l'invention est avantageusement autoadhérente.

Contrairement à ce que l'on aurait pu attendre, vu la fragilité de ces non tissés spécifiques, on constate que l'on peut assembler ces derniers sans les déstructurer ou les fragiliser encore plus, et sans détériorer leurs propriétés d'extensibilité et d'autoadhérence.

La demande US 2003/040691 décrit un bandage élastique comportant une bande non élastique non tissé absorbante, un voile non-tissé perméable à l'air non-élastique, et un matériau élastomère filé par fusion disposée entre les deux nappes.

Le matériau élastomère filé par fusion peut comprendre une pluralité de filaments en élastomère filé par fusion alignés dans la distribution sensiblement parallèle à une direction de la machine, les filaments fixés à un côté de la nappe non tissée non élastique et perméable à l'air sur un côté de la nappe non élastique non tissé absorbant.

La demande US 2011/208101 se rapporte à un pansement de compression destiné à fournir des propriétés de transport de l'humidité à une surface de contact.

Le pansement de compression est composé d'au moins une couche de tissu de transport de l'humidité constituée de fibres élastiques qui présentent un taux d'élasticité suffisant pour appliquer une force de compression sur une partie du corps. Le pansement compressif permet également de gérer des fluides et comprend une couche réservoir d'absorption qui est appliquée à la surface extérieure de la toile de transport de l'humidité. Cette couche réservoir absorbant peut être retirée en fonction des besoins sans perturber la force de compression appliquée à une partie du corps.

Un pansement poreux auto-adhésif élastique a été décrit dans le brevet US 5 393 339 comme moyen de compression enroulé autour d'une plaie et moyen d'absorption des fluides et exsudats de la plaie. Une forme de réalisation préférée du pansement est constituée d'un substrat élastique auto-adhésif qui ne colle pas aux vêtements fixé à une couche absorbante.

La présente invention est donc relative à une bande de contention comprenant deux non tissés qui ont été obtenus à partir de fibres courtes conjuguées, les deux non tissés étant assemblés l'un à l'autre. La bande peut être élastique et autoadhérente sans contenir du latex. Avantageusement, la bande peut contenir moins de 0.01% en poids de latex ou d'un adhésif de faible adhérence, tout en étant auto-adhérente.

Les deux non tissés peuvent être assemblés sur l'ensemble de leur surface; la valeur et la forme de leurs surfaces peuvent être identiques ou différentes. On préfère que les deux non tissés soient de surface et de forme identiques.

Les fibres sont avantageusement frisées de façon uniforme dans le sens de l'épaisseur des non tissés, et présentent un rayon de courbure moyen de préférence compris entre 10 et 200 micromètres. Le nombre de fibres frisées à la surface du non-tissé est avantageusement supérieur à 10 fibres frisées/cm².

Les deux non tissés ont de préférence indépendamment l'un de l'autre un grammage compris entre 70 g/m² et 300 g/m². Ils ont de préférence le même grammage.

Dans un mode de réalisation particulier, l'invention a pour objet une bande de contention comprenant au moins deux non tissés, de préférence deux non tissés, de fibres frisées obtenues à partir de fibres courtes conjuguées, dans laquelle
- les non tissés sont assemblés l'un à l'autre et ont indépendamment l'un de l'autre un grammage compris entre 70 g/m² et 300 g/m²,
- lesdites fibres sont frisées de façon uniforme dans le sens de l'épaisseur des non tissés, et présentent un rayon de courbure moyen compris entre 10 et 200 micromètres, et
- le nombre de fibres frisées à la surface de chacun des non-tissés est supérieur à 10 fibres frisées/cm².

Une variante de la présente invention est relative à une bande de contention qui comprend au moins une couche supplémentaire entre les deux non tissés.

Des non tissés utilisables dans le cadre de la présente invention sont décrits dans la demande de brevet WO 2008/015972.

De façon générale, les fibres qui ont été utilisées pour fabriquer le non tissé sont de préférence conjuguées, de nature polymérique, et non continues (courtes).

Les fibres conjuguées au sens de l'invention sont des fibres «à frisabilité latente» dotées d'une structure asymétrique ou stratifiée, qui ont la propriété de friser sous l'effet d'un chauffage. Elles doivent cette propriété à la différence de coefficient de contraction thermique des polymères qui les constituent.

Ces fibres sont avantageusement constituées d'au moins deux polymères qui présentent un coefficient de contraction thermique différent. Ces polymères ont ordinairement des points de ramollissement ou des points de fusion différents. Ils peuvent être choisis parmi les polymères thermoplastiques comme par exemple : les polymères oléfiniques (notamment les polymères de polyoléfines C₂₋₄ tels que les polyéthylènes et polypropylènes basse, moyenne et haute densité), les polymères acryliques (notamment les polymères acrylonitriliques à unités acrylonitrile tels que les copolymères acrylonitrile/chlorure de vinyle), les polymères vinylacétaliques (notamment les polymères de polyvinylacétal), les polymères chlororovinyliques (notamment les polychlorures de vinyle, les copolymères chlorure de vinyle/acétate de vinyle et les copolymères chlorure de vinyle/acrylonitrile), les polymères chlorovinylidéniques (notamment les copolymères chlorure de vinylidène/chlorure de vinyle et les copolymères chlorure de vinylidène/acétate de vinyle), les polymères styréniques (notamment les polystyrènes résistants à la chaleur), les polymères polyesters (notamment les polymères de polyalkylène C₂₋₄ arylates tels que les polymères de polyéthylène téréphtalate, de polytriméthylène téréphtalate, de polybutylène téréphtalate et de polyéthylène naphtalate), les polymères polyamides (notamment les polymères polyamides aliphatiques tels que les polyamides 6, 6-6, 11, 12, 6-10 et 6-12, les polymères polyamides semi-aromatiques, les polymères polyamides aromatiques tels que le polyphénylène isophtalamide, le polyhexaméthylène téréphtalamide et le polyparaphénylène téréphtalamide), les polymères polycarbonates (notamment les polycarbonates de type bisphénol A), les polymères de polyparaphénylène benzobisoxazole, les polymères de polysulfure de phénylène, les polymères polyuréthanes, les polymères cellulosiques (notamment les esters de cellulose), etc. Ces polymères thermoplastiques peuvent éventuellement contenir d'autres unités copolymérisables.

Lorsque le chauffage des fibres est réalisé avec de la vapeur à haute température, selon le mode préféré de réalisation du non tissé, on préfère des polymères non adhésifs sous chaleur humide (ou des polymères hydrophobes ou non hydrosolubles résistants à la chaleur) de point de ramollissement ou de point de fusion supérieur ou égal à 100°C, comme par exemple les polymères polypropyléniques, les polymères polyesters et les polymères polyamides. Ces polymères permettent d'éviter le collage des fibres par fusion ou le ramollissement des fibres. On préfère tout particulièrement les polymères polyesters aromatiques et les polymères polyamides, pour leur excellente stabilité, leur résistance à la chaleur et leur aptitude à former des fibres.

Selon un mode préféré de la présente invention les fibres utilisées sont bicomposants. Les fibres bicomposants peuvent être composées de polymères de la même famille chimique ou de polymères de familles chimiques différentes, pourvu qu'ils aient des coefficients de contraction thermiques différents.

Dans un mode de mise en oeuvre, les fibres courtes conjuguées sont bicomposants, les deux composants les constituant étant des polymères qui présentent un point de ramollissement supérieur ou égal à 100°C, lesdits polymères étant choisis parmi les polymères polypropyléniques, les polymères polyesters et/ou les polymères polyamides, et de préférence sont deux polymères polyesters aromatiques différents.

On préfère que les fibres bicomposants soient constituées de deux polymères de la même famille chimique : par exemple d'un homopolymère et d'un copolymère. On peut en effet abaisser le taux de cristallinité de l'homopolymère, voire le rendre amorphe, ou abaisser son point de fusion ou son point de ramollissement, en copolymérisant le monomère avec un autre. L'écart de point de fusion ou de point de ramollissement des deux polymères peut être par exemple de l'ordre de 5 à 150°C, préférentiellement de 50 à 130°C, et plus préférentiellement de 70 à 120°C. La proportion de monomère copolymérisable, rapportée à la quantité totale de monomères, est par exemple de l'ordre de 1 à 50 % en moles, préférentiellement de 2 à 40 % en moles, et plus préférentiellement de 3 à 30 % en moles (particulièrement de 5 à 20 % en moles). Le rapport pondéral de l'homopolymère et du copolymère peut être choisi en fonction de la structure des fibres; il est par exemple, en termes de rapport homopolymère (A)/copolymère (B), de l'ordre de 90/10 à 10/90, préférentiellement de 70/30 à 30/70, et plus préférentiellement de 60/40 à 40/60. Dans un mode de réalisation préféré, les fibres bicomposants sont constituées de deux polymères polyesters aromatiques et en particulier de l'association d'un homopolymère de polyalkylène arylate (a) et d'un copolymère de polyalkylène arylate (b). L'homopolymère de polyalkylène arylate (a) peut être un homopolymère d'un acide dicarboxylique aromatique (notamment un acide dicarboxylique aromatique symétrique tel que l'acide téréphtalique ou l'acide naphtalène-2,6-dicarboxylique) et d'un composant alcane-diol (notamment l'éthylène-glycol ou le butylène-glycol). On utilise par exemple un polymère de la série des polyalkylène téréphtalates tel que le polyéthylène téréphtalate (PET) ou le polybutylène téréphtalate (PBT), et ordinairement un PET de viscosité intrinsèque de l'ordre de 0,6 à 0,7 servant à la fabrication des fibres de PET ordinaires. Le copolymère de polyalkylène arylate (b) peut être obtenu à partir d'un premier monomère servant à la préparation de l'homopolymère de polyalkylène arylate (a), et d'un deuxième monomère choisi parmi un acide dicarboxylique tel qu'un acide dicarboxylique aromatique asymétrique, un acide dicarboxylique alicyclique, un acide dicarboxylique aliphatique, un composant alcane-diol de chaîne plus longue que l'alcane-diol du polymère de polyalkylène arylate (a), et/ou un diol porteur d'une liaison éther.

Il est possible d'utiliser un seul ou d'associer plusieurs de ces deuxièmes monomères. Parmi ces composants, on utilise de préférence :
- un acide dicarboxylique aromatique asymétrique, notamment l'acide isophtalique, l'acide phtalique ou l'acide 5-sulfoisophtalique de sodium,
- ou un acide dicarboxylique aliphatique, notamment un acide dicarboxylique aliphatique C₁₋₁₂ tel que l'acide adipique,
- un alcane-diol, notamment le 1,3-propane-diol, le 1,4-butane-diol, le 1,6-hexane-diol ou le néopentylglycol,
- un polyoxyalkylène-glycol, notamment le diéthylène-glycol, le triéthylène-glycol, le polyéthylène-glycol ou le polytétraméthylène-glycol.

Parmi eux, on choisit de préférence notamment un acide dicarboxylique aromatique asymétrique tel que l'acide isophtalique, et un polyoxyalkylène-glycol tel que le diéthylène-glycol. Le copolymère de polyalkylène arylate (b) peut éventuellement être un élastomère à segments durs d'alkylène arylate (éthylène téréphtalate, butylène téréphtalate) et à segments souples par exemple de (poly)oxyalkylène-glycol. Dans le copolymère de polyalkylène arylate (b), la proportion de composant acide dicarboxylique destiné à abaisser le point de fusion ou le point de ramollissement rapportée à la quantité totale de composant acide dicarboxylique est par exemple de l'ordre de 1 à 50 % en moles, préférentiellement de 5 à 50 % en moles, et plus préférentiellement de 15 à 40 % en moles. La proportion de composant diol destiné à abaisser le point de fusion ou le point de ramollissement rapportée à la quantité totale de composant diol, est par exemple d'au plus 30 % en moles, et préférentiellement d'au plus 10 % en moles, par exemple de l'ordre de 0,1 à 10 % en moles.

La section transversale (perpendiculaire au sens de la longueur des fibres) des fibres bicomposants n'est pas limitée à la forme ronde (la forme ordinaire des fibres pleines) et aux formes modifiées (aplatie, elliptique, polygonale, 3- à 14-foliée, en T, en H, en V, en «os à chien» (en i), etc.), mais peut être aussi une section creuse. Toutefois, on choisit usuellement la section ronde.

Pour la structure transversale des fibres bicomposants, on peut citer les structures phasées formées par une pluralité de polymères, comme par exemple les structures de types coeur-écorce, îles et mer, mélangé, parallèle (côte-à-côte ou laminé multicouche), radial (laminé radial), radial creux, à blocs ou aléatoire. Parmi ces structures, on préfère, pour un développement plus spontané du frisage thermique, une structure de type coeur-écorce excentrique ou de type parallèle. Dans le cas de fibres bicomposants de type coeur-écorce et par exemple de type coeur-écorce excentrique, l'âme peut être constituée d'un polymère de la famille de l'alcool vinylique tel qu'un copolymère éthylène/alcool vinylique ou un alcool polyvinylique, ou d'un polymère thermoplastique de point de fusion ou de point de ramollissement bas, par exemple un polystyrène ou un polyéthylène basse densité, pourvu qu'elle autorise le frisage par le fait d'avoir un écart de coefficient de contraction thermique avec le polymère constituant l'écorce.

Dans un mode de réalisation particulier, les fibres bicomposants ont une structure de type côte à côte et sont constituées d'un premier polymère qui est un polyéthylène téréphtalate, et d'un second polymère qui est un copolymère d'un alkylène arylate avec l'acide isophtalique et/ou du diéthylène glycol.

Le titre moyen des fibres courtes conjuguées, notamment bicomposants, peut être par exemple compris entre 0,1 à 50 dtex, préférentiellement entre 0,5 et 10 dtex, et plus préférentiellement entre 1 et 5 dtex (particulièrement entre 1,5 et 3 dtex). Si le titre est trop fin, non seulement les fibres sont difficiles à fabriquer mais elles risquent de manquer de résistance. De plus, à l'étape de frisage, il est difficile d'obtenir de belles frisures en serpentin. Si le titre est trop gros, les fibres deviennent raides et rendent difficile le développement d'un frisage suffisant.

La longueur moyenne des fibres courtes conjuguées avant le frisage peut être par exemple comprise entre 10 et 100 mm, préférentiellement entre 20 et 80 mm, et plus préférentiellement entre 25 et 75 mm (particulièrement entre 40 et 60 mm). Si les fibres sont trop courtes, outre la difficulté de former le voile de fibres, l'enchevêtrement des fibres est insuffisant à l'étape de frisage et il est difficile de garantir de bonnes propriétés de résistance et d'extensibilité. Si les fibres sont trop longues, non seulement il devient difficile de former un voile de fibres de grammage uniforme, mais les fibres s'enchevêtrent excessivement lors de la formation du voile, au point de se gêner mutuellement au moment du frisage et d'empêcher le développement de l'extensibilité. De plus, dans l'invention, le choix de la longueur de fibre dans la plage précitée permet à une partie des fibres frisées à la surface du non-tissé d'émerger modérément de ladite surface du non-tissé et ainsi d'améliorer l'auto-adhésivité du non-tissé qui sera évoquée plus loin.

Dans un mode de réalisation, le titre moyen des fibres courtes conjuguées est compris entre 1 et 5 dtex, de préférence entre 1,5 et 3 dtex, et la longueur moyenne des fibres courtes conjuguées est comprise entre 10 à 100 mm, et de préférence entre 40 et 60 mm.

L'application d'un traitement thermique à ces fibres conjuguées a pour effet de développer le frisage et de leur imprimer des frisures en relief ayant la forme de serpentins (en spirale ou en «ressort à boudin»). Le rayon de courbure moyen des fibres frisées au sens de l'invention correspond au rayon de courbure moyen des cercles formés par les boucles des serpentins des fibres frisées ; il peut compris entre 10 et 200 microns, par exemple entre 10 et 250 microns, de préférence entre 20 et 200 microns, préférentiellement entre 50 et 160 microns, et plus préférentiellement entre 70 et 130 microns.

Le rayon de courbure moyen des fibres frisées peut être déterminé par microscopie électronique selon la méthode suivante. On prend une micrographie (grossissement x100) d'une section de non-tissé au microscope électronique à balayage (MEB). Parmi les fibres apparaissant sur le cliché, on sélectionne les fibres formant au moins 1 tour de spirale (serpentin) et on détermine leur rayon de courbure comme le rayon du cercle tracé le long de la spirale (rayon du cercle quand on observe la fibre frisée dans le sens de l'axe du serpentin). Quand la fibre dessine une spirale elliptique, le rayon de courbure est déterminé comme la demi-somme des grand et petit diamètres de l'ellipse. Afin d'exclure les fibres ayant développé un frisage en serpentin insuffisant et les fibres apparaissant elliptiques à cause d'une observation oblique de la spirale, on s'est limité aux fibres elliptiques de rapport entre grand et petit diamètres compris entre 0,8 et 1,2. On réalise la mesure sur l'image MEB d'une section arbitraire de non tissé et on détermine la moyenne sur une population de fibres n=100.

Lorsque le frisage est réalisé par de la vapeur à haute température, le non-tissé selon l'invention a pour caractéristique que le frisage des fibres conjuguées orientées à peu près parallèlement au sens planaire est développé de façon à peu près uniforme dans le sens de l'épaisseur. Dans une section de non tissé prise dans le sens de l'épaisseur, parmi les domaines délimités par un partage en trois parts égales dans le sens de l'épaisseur, le nombre de fibres formant au moins 1 tour de frisure spiralée est par exemple, dans la partie centrale (couche interne), de 5 à 50 par 5 mm (longueur en sens planaire) et 0,2 mm (épaisseur), préférentiellement de 10 à 50 par 5 mm (planaire) et 0,2 mm (épaisseur), et plus préférentiellement de 20 à 50 par 5 mm (planaire) et 0,2 mm (épaisseur).

Comme la majeure partie des fibres frisées ont leur axe orienté dans le sens planaire et que le nombre de frisures est uniforme dans le sens de l'épaisseur, le non-tissé manifeste une haute extensibilité (sans contenir de caoutchouc ou d'élastomère), et une bonne résistance opérationnelle (sans contenir d'adhésifs).

Dans la présente description, par «domaines délimités par un partage en trois parts égales dans le sens de l'épaisseur», on entend les différents domaines obtenus quand on coupe le non-tissé en trois tranches égales orientées perpendiculairement à l'épaisseur.

Dans le non-tissé, l'uniformité du frisage dans le sens de l'épaisseur peut être définie par le ratio d'incurvation de fibre. Par «ratio d'incurvation de fibre», on entend le rapport L2/L1 de la longueur de la fibre étirée bidimentionnellement L2 à la distance L1 des deux extrémités de la fibre à l'état frisé. Ce ratio d'incurvation de fibre (en particulier dans le domaine central dans le sens de l'épaisseur) est par exemple de l'ordre d'au moins 1,3 (par exemple de 1,35 à 5), préférentiellement de 1,4 à 4 (par exemple de 1,5 à 3,5), et plus préférentiellement de 1,6 à 3 (particulièrement de 1,8 à 2,5).

Lorsque le ratio d'incurvation de fibre est mesuré sur la base de micrographies électroniques de sections du non-tissé, la longueur de fibre L2 ne correspond pas à la longueur de la fibre qui serait obtenue si on étirait et rectilinéarisait la fibre frisée tridimensionnellement. Elle correspond à la longueur de fibre sur cliché qui est obtenue quand on étire et rectilinéarise la fibre apparaissant frisée bidimensionnellement sur le cliché. Autrement dit, la longueur de fibre sur cliché qui est mesurée selon l'invention est inférieure à la longueur de fibre réelle.

Lorsque le développement du frisage est à peu près uniforme dans le sens de l'épaisseur, le ratio d'incurvation de fibre est également uniforme dans le sens de l'épaisseur. L'uniformité du ratio d'incurvation de fibre peut être évaluée en comparant, dans une section prise dans le sens de l'épaisseur, les ratios d'incurvation de fibre obtenus dans les différentes couches délimitées par un partage en trois parts égales dans le sens de l'épaisseur. Ainsi, dans une section prise dans le sens de l'épaisseur, les ratios d'incurvation de fibre obtenus dans les différents domaines délimités par le partage en trois parts égales dans le sens de l'épaisseur se situent tous dans la plage précitée, et le rapport de la valeur minimale à la valeur maximale du ratio d'incurvation de fibre dans les différents domaines (rapport du domaine où le ratio d'incurvation de fibre est minimal au domaine où il est maximal) est par exemple de l'ordre d'au moins 75 % (par exemple de 75 à 100 %), préférentiellement de 80 à 99 %, et plus préférentiellement de 82 à 98 % (particulièrement de 85 à 97 %).

Selon un mode de réalisation, le non tissé présente dans une section prise dans le sens de l'épaisseur, un ratio d'incurvation de fibre supérieur ou égal à 1,3 dans chacun des domaines délimités par un partage en trois parts égales dans le sens de l'épaisseur, et le rapport de la valeur minimale à la valeur maximale du ratio d'incurvation de fibre dans les différents domaines est supérieur à 75 %.

Comme méthode concrète de mesure du ratio d'incurvation de fibre et de son uniformité, on peut appliquer la méthode qui consiste à prendre une micrographie de la section du non-tissé au microscope électronique et à mesurer le ratio d'incurvation de fibre sur des domaines choisis au sein des différents domaines du partage en trois parts égales dans le sens de l'épaisseur. La mesure est réalisée, dans chacune des couches supérieure (domaine recto), interne (domaine central) et inférieure (domaine verso), sur des domaines qui, dans le sens de la longueur, font au moins 2 mm, et dans le sens de l'épaisseur, sont positionnés près du centre de chaque couche et ont la même épaisseur d'un domaine à l'autre. De plus, ces domaines de mesure sont parallèles dans le sens de l'épaisseur, et sont définis de telle sorte que chacun renferme au moins 100 fragments de fibres autorisant la mesure de leur ratio d'incurvation (de l'ordre préférentiellement d'au moins 300, et plus préférentiellement de 500 à 1000). Après avoir défini ces domaines de mesure, on mesure le ratio d'incurvation de fibre de toutes les fibres situées dans le domaine et on calcule la valeur moyenne sur chaque domaine de mesure, puis on calcule l'uniformité du ratio d'incurvation de fibre en comparant le domaine montrant la valeur moyenne la plus grande et le domaine montrant la valeur moyenne la plus petite.

La mesure du ratio d'incurvation de fibre et de son uniformité peut être effectuée selon la méthodologie suivante. On prend une micrographie (grossissement x100) d'une section de non-tissé au microscope électronique et, dans une partie où apparaissaient les fibres sur le cliché, on partage l'épaisseur en trois domaines égaux (couches recto, interne et verso), et près du centre de chaque domaine, on définit des domaines de mesure d'au moins 2 mm dans le sens de la longueur et contenant au moins 500 fragments de fibres mesurables. Sur ces domaines, on mesure d'une part la distance inter-extrémités (distance la plus courte) entre les deux extrémités de la fibre et d'autre part la longueur de fibre (longueur de la fibre sur cliché).

Précisément, quand une extrémité de fibre émerge à la surface du non-tissé, elle est retenue telle quelle comme extrémité de mesure de la distance inter-extrémités; quand une extrémité de fibre plonge dans le non-tissé, on retient la partie limite de plongée dans le non-tissé (extrémité sur cliché) comme extrémité de mesure de la distance inter-extrémités.

Parmi les fibres imagées, on exclut de la mesure celles sur lesquelles on ne peut isoler une continuité d'au moins 100 µm. On calcule le ratio d'incurvation de fibre comme le rapport L2/L1 de la longueur de fibre L2 à la distance inter-extrémités L1 des fibres. Puis on calcule la moyenne sur chacune des couches recto, interne et verso du partage en trois parts égales dans le sens de l'épaisseur. On calcule enfin l'uniformité du ratio d'incurvation de fibre dans le sens de l'épaisseur à partir du rapport de ses valeurs maximale et minimale dans les différentes couches.

Le principe de la méthode de mesure de la longueur de fibre est illustré sur les figures 4-a et 4-b de la demande de brevet WO 2008/015972.

La Fig.4-(a) illustre le cas d'une fibre dont une extrémité émerge à la surface et l'autre extrémité plonge dans le non-tissé. La distance inter-extrémités L1 est ici la distance d'une extrémité de la fibre jusqu'à la partie limite de plongée dans le non-tissé. D'autre part, la longueur de fibre L2 est la longueur obtenue quand on étire bidimensionnellement, sur le cliché, la partie de la fibre observable (partie allant de l'extrémité de la fibre jusqu'à la partie de plongée dans le non-tissé).

La Fig.4-(b) illustre le cas d'une fibre dont les deux extrémités plongent dans le non-tissé. La distance inter-extrémités L1 est ici la distance des deux extrémités de la partie émergeant à la surface du non-tissé (extrémités sur cliché). D'autre part, la longueur de fibre L2 est la longueur obtenue quand on étire bidimensionnellement, sur le cliché, la fibre dans la partie émergeant à la surface du non-tissé.

Pour des fibres frisées en forme de serpentin, le pas moyen du serpentin est par exemple de l'ordre de 0,03 à 0,5 mm, préférentiellement de 0,03 à 0,3 mm, et plus préférentiellement de 0,05 à 0,2 mm.

Le non-tissé peut aussi contenir des fibres qui ne sont pas des fibres bicomposants. Parmi ces fibres additionnelles mono-composants, on peut par exemple citer les fibres de polymères déjà cités précédemment, mais aussi les fibres cellulosiques comme par exemple les fibres naturelles (laine de bois, laine de mouton, soie, chanvre), les fibres semi-synthétiques (notamment les fibres d'acétate telles que les fibres de triacétate) ou les fibres régénérées (rayonne, lyocell). Le titre moyen et la longueur moyenne des fibres mono-composants sont de préférence identiques à ceux des fibres bicomposants. On peut utiliser une seule espèce ou associer plusieurs espèces de ces fibres mono-composants. Parmi ces fibres mono-composants, la préférence va notamment aux fibres régénérées telles que les fibres de rayonne, aux fibres semi-synthétiques telles que les fibres d'acétate, aux fibres polyoléfiniques telles que les fibres de polypropylène ou de polyéthylène, aux fibres polyesters et aux fibres polyamides.

On préfère associer à des fibres bicomposants d'une famille chimique (par exemple de polyester) des fibres mono-composants de la même famille chimique.

Le rapport pondéral entre les fibres bicomposants et les fibres mono-composants est par exemple de l'ordre de 80/20 à 100/0 (par exemple de 80/20 à 99/1), préférentiellement de 90/10 à 100/0, et plus préférentiellement de 95/5 à 100/0.

Le non tissé qui compose la bande de l'invention est avantageusement dépourvu de fibres élastomériques. De telles fibres élastomériques sont généralement des filaments ou des fibres longues obtenus à partir de matériaux thermoplastiques tels que le polyuréthane, le polyamide, les copolymères du styrène, ou le polyester. Elles sont généralement obtenues par extrusion soufflage (« meltblown » en anglais) et ont généralement une longueur supérieure à 100 mm. Les non tissés sont avantageusement dépourvus de fibres longues disposées dans le sens longitudinal de la bande.

Le non-tissé peut aussi contenir des additifs tels que des agents stabilisants, des filtres UV, des photostabilisants, des antioxydants, des antibactériens, des désodorisants, des parfums, des colorants, des charges, des agents antistatiques, des retardateurs de flamme, des plastifiants, des lubrifiants, ou des retardateurs de cristallisation. On peut utiliser un seul ou plusieurs de ces additifs. Ces additifs peuvent aussi bien être supportés à la surface des fibres que contenus à l'intérieur des fibres.

Afin de pouvoir obtenir une bande de contention aux propriétés recherchées, on choisira, parmi les non tissés réalisés à partir des fibres et des polymères précédemment décrits, deux non tissés qui présentent in dépendamment l'un de l'autre un grammage compris entre 70 et 300 g/m², de préférence compris entre 80 et 200 g/m², et de préférence encore entre 90 et 150 g/m². Le grammage peut être mesuré selon la norme EN 9073-1.

Le grammage total des deux non tissés est par exemple supérieur à 200 g/m², voir même supérieur à 220 g/m². Dans un mode de mise en oeuvre, le grammage total des deux non tissés est compris entre 220 et 300 g/m².

Un grammage trop faible rend impossible l'assemblage car le produit trop fragile risque d'être détruit lors de l'assemblage, et un grammage trop élevé ne permet pas d'obtenir le compromis souhaité entre les caractéristiques d'extensibilité, de déchirabilité et d'autoadhérence.

Les autres propriétés mécaniques du non tissé seront de préférence les suivantes.

L'épaisseur du non tissé sera avantageusement comprise entre 0,25 et 5 mm, de préférence entre 0,4 et 2,5 mm et tout particulièrement entre 0,5 et 1,5 mm. L'épaisseur peut être mesurée selon la norme EN 9073-2.

L'élongation du non-tissé, c'est à dire son allongement à la rupture sera dans le sens longitudinal avantageusement compris entre 60 et 200% et de préférence entre 90 et 130 %, et dans le sens transversal compris entre 70 et 200 % et de préférence entre 60 et 160 %. L'élongation longitudinale et transversale peuvent être mesurées selon la norme EN 9073-3. Le test de cette norme consiste à mesurer l'allongement à la rupture, exprimé en pourcentage, qui correspond à la valeur de l'élongation. Les conditions de test dans le sens longitudinal sont les suivantes.

On soumet un échantillon du matériau à tester (par exemple le non tissé) de 300 mm de longueur et de 50 mm de largueur à un test de traction à l'aide d'un dynamomètre électronique dans lequel la traverse se déplace à une vitesse de 100 mm/mn. L'espace entre les mors est réglé à 200 mm et la largeur est celle de l'éprouvette soit 50 mm. Le dynamomètre s'arrête automatiquement quand l'échantillon subit sa rupture et l'appareillage enregistre l'allongement à la rupture. On reproduit le test sur trois échantillons et on prend la valeur moyenne.

Dans le sens transversal la mesure est réalisée de façon identique en adaptant la longueur entre les mors à la largeur du matériau à tester; par exemple avec un matériau de 10 cm de large, la longueur de l'échantillon entre les mors est de 6 cm.

L'élasticité du non tissé, telle que définie dans la norme EN 14704-1 c'est à dire sa récupération élastique, après un allongement de 30% est de préférence supérieure ou égale à 70% (par exemple comprise entre 70 et 100%) et de préférence comprise entre 80 et 95 %.

Le principe de la norme EN 14704-1 est basé sur la mesure de la récupération élastique est le suivant. Les conditions de la mesure sont les suivantes.

Une éprouvette (par exemple de bande de contention ou de non tissé) de 50 mm de largeur et de 200 mm de longueur est insérée dans les mors d'un dynamomètre électronique, lequel va effectuer une série de 5 cycles, jusqu'à un allongement de 30%, de traction «charge-décharge» à une vitesse de 100 mm/mn. L'allongement recouvré obtenu au cinquième cycle, exprimé en pourcentage, tel que défini dans la norme est automatiquement mesuré par le dynamomètre. La mesure de la récupération élastique, exprimée en pourcentage, est calculée selon la formule définie dans la norme sur la base de cet allongement recouvré. L'opération est répétée sur trois éprouvettes et on prend la valeur moyenne.

Dans le cadre de la présente invention on considère qu'un matériau est élastique si sa récupération élastique est supérieure ou égale à 70%.

L'autoadhérence de la bande selon l'invention est obtenue grâce à la présence de nombreuses fibres à l'état partiellement libre à la surface des non tissés, les fibres de surface s'enchevêtrant mutuellement au moment de la superposition de la bande sur elle-même. Pour obtenir cette propriété d'autoadhérence sans altérer les propriétés de déchirabilité et d'extensibilité, le nombre de fibres frisées, notamment en forme de serpentin ou de boucle, à la surface du non tissé, est avantageusement supérieur à 10 fibres frisées/cm², et de préférence compris entre 10 et 50 fibres frisées/cm². Pour la réalisation d'une bande de contention, on préférera un nombre de fibres frisées à la surface du non tissé compris entre 10 à 35 fibres frisées/cm².

Le nombre de fibres frisées à la surface du non tissé peut être déterminé comme suit.

On prend une micrographie (grossissement x100) de la surface du non-tissé au microscope électronique, et on compte le nombre de fibres frisées (fibres faisant au moins un tour de spirale en boucle ou de serpentins formés à la surface du non-tissé), sur une aire unitaire de 1 cm² de surface de fibres imagées. La mesure peut être effectuée en cinq endroits arbitraires, et on calcule le nombre moyen de fibres bouclées arrondie à l'unité la plus proche.

La bande de contention selon l'invention est réalisée par l'assemblage de deux non tissés choisis parmi ceux tels que précédemment définis. Cet assemblage sera réalisé de manière à ne pas altérer les propriétés d'autoadhérence et d'extensibilité des deux non tissés, et à garantir l'absence de délaminage du produit au cours du temps.

La caractérisation de l'autoadhérence des non tissés ou de la bande est évaluée par la mesure de la force de pelage d'un échantillon de non tissé ou de bande replié sur lui-même. Cette force de pelage varie entre 0,02 et 0,5 N/cm et de préférence entre 0,025 et 0,1 N/cm.

Le test de caractérisation de l'autoadhérence peut consister à mesurer la force de pelage à 180° d'un échantillon de matériau à l'aide d'un dynamomètre électronique. Cette force de pelage représente la valeur de l'autoadhérence du matériau. Les conditions de réalisation de la mesure sont avantageusement les suivantes.

Un échantillon de 60 cm de longueur et de 5 cm de largueur est replié sur lui-même en laissant libre les extrémités qui serviront à le fixer dans les mors du dynamomètre électronique. Les deux faces sont mises en contact sous la pression d'un poids équivalent à 1 kgf/cm. On effectue le pelage en réglant le dynamomètre à une vitesse de 300 mm/mn. Le dynamomètre donne directement la force de pelage, exprimée en N/cm. On reproduit le test sur trois échantillons et on prend la valeur moyenne.

La bande de contention selon l'invention est réalisée par l'assemblage de deux non tissés choisis parmi ceux tels que précédemment définis.

Par assemblage, on entend tout moyen permettant de lier les deux non tissés ensemble, si bien que la simple superposition des deux non tissés autoadhérents ne peut être considérée comme un assemblage. On pourra - afin d'obtenir les propriétés de contention recherchées -combiner deux non tissés identiques ou différents. Pour le traitement des ulcères de jambes, on préférera tout particulièrement combiner deux non tissés identiques présentant un grammage compris entre 90 et 150 g/m².

Dans un mode de réalisation particulier, la bande de contention de l'invention comprend deux non tissés, de préférence identiques, constitués de fibres bicomposants côte à côte à base de polymères polyesters aromatiques, chaque non tissé ayant un grammage compris entre 90 et 150 g/m² et le nombre de fibres frisées à la surface de chaque non tissé est compris entre 10 et 35 fibres frisées/cm².

Des technologies textiles variées, comme par exemple la couture, l'aiguilletage, la soudure par ultrasons, le complexage ou la fixation à l'aide d'un adhésif peuvent être utilisées pour réaliser l'assemblage des deux non tissés entre eux, ou l'assemblage des deux non tissés avec une couche supplémentaire. Ces technologies seront choisies en fonction de la nature des matériaux à assembler, en particulier leur résistance à la température et leur résistance mécanique.

Les non tissés sont de préférence assemblés par aiguilletage, avec un adhésif ou par ultra-sons. On préférera tout particulièrement un assemblage par aiguilletage. Dans un mode de réalisation, les deux non tissés sont aiguilletés avec une couche supplémentaire qui est une ouate présentant une épaisseur comprise entre 2 et 3 mm.

Afin de garantir l'absence de délaminage du produit on s'assurera que la force de délaminage entre les non tissés et/ou la couche supplémentaire éventuelle soit supérieure à 10 cN/cm, et de préférence supérieure à 25 cN/cm.

Le principe de la mesure de délaminage est basé sur la méthode communément appelée décomplexage en T dans laquelle on mesure la force nécessaire pour délaminer les matériaux composant la bande de contention. La mesure de cette force de délaminage peut être réalisée selon le protocole suivant. On découpe une éprouvette de bande de contention de 50 mm de largeur et 300 mm de longueur. On délamine manuellement l'extrémité de cette éprouvette sur une longueur de 1 à 3 cm de manière à fixer chaque extrémité délaminée de la bande dans les mors d'un dynamomètre. La mesure est réalisée de telle sorte que l'on a un angle de 90° entre la bande de contention et l'extrémité de la bande préalablement délaminée. La force de délaminage est mesurée à l'aide du dynamomètre électronique dans lequel la traverse est mobile et se déplace à la vitesse de 300 mm/mn. Le dynamomètre enregistre directement cette force mesurée qui est exprimée en cN/cm. On reproduit le test sur 3 éprouvettes et on prend la valeur moyenne.

Selon une variante de la présente invention, on pourra intercaler entre les deux non tissés une couche supplémentaire choisie parmi les matériaux textiles, les matériaux alvéolaires, les films ou leurs combinaisons. Cette couche supplémentaire permet d'améliorer si nécessaire, selon la pathologie à traiter ou les utilisations envisagées, les propriétés de la bande autoadhérente élastique obtenue par assemblage des deux non tissés, par exemple en adaptant ses capacités d'absorption, d'amortissement, de conformabilité, de rigidité, ou d'occlusivité.

Parmi les matériaux textiles, on pourra utiliser des matériaux à base de fibres synthétiques ou naturelles. On peut citer les tissés, les non tissés, les tricots, les tricots 3D et leurs combinaisons.

Parmi les non tissés inélastiques on préférera utiliser des non tissés absorbants qui présentent une épaisseur supérieure à 1,8 mm, et de préférence comprise entre 1,8 et 4 mm, et en particulier comprise entre 2 et 3 mm.

Parmi de tels non tissés on peut citer les non tissés à base de fibres absorbantes, tels que les compresses absorbantes utilisées dans le domaine des pansements et les ouates, ou un non tissé choisi parmi ceux décrits dans la demande de brevet WO 2008/015972.

On préférera l'utilisation de matériaux qui permettent d'améliorer les propriétés d'amortissement ou d'absorption, et tout particulièrement les matériaux alvéolaires, les tricots 3D et les non tissés. On choisira de préférence les mousses polyuréthane hydrophiles, et les non tissés absorbants tels que les compresses absorbantes et les ouates. A titre d'exemple, ces ouates peuvent être constituées de fibres de viscoses, de polyester, coton ou de rayonne. De telles ouates sont par exemple les produits commercialisés par les sociétés URGO Limited, ACTIVA ou SMITH et NEPHEW respectivement sous les dénominations K-SOFT®, FLEXI-BAN® et SOFT-BAN®.

Dans le cadre de la présente invention, on préférera tout particulièrement la ouate K-SOFT® qui est constituée d'un mélange de 60 % de fibres viscose et 40 % de fibres polyester et qui présente une épaisseur de 2,5 mm et un grammage de 75 g/m².

Parmi les matériaux alvéolaires, on peut citer les mousses hydrophobes ou hydrophiles par exemple à base de polyuréthane ou à base d'oléfines. On préférera en particulier, dans le cas du traitement des ulcères de jambes, des mousses hydrophiles absorbantes comme par exemple la mousse commercialisée sous la référence MCF.03 par la société Advanced Medical Solutions (AMS). Comme mousse non absorbante on peut citer par exemple les mousses à base d'oléfines commercialisées par la société ALVEO sous la dénomination Alveolit®.

La couche supplémentaire peut être une mousse de polyuréthane hydrophile ou une ouate.

Comme films on peut utiliser tout film polymérique souple par exemple à base de polyuréthane, polyoléfine, polyamide, polyester ou polychlorure de vinyle.

Parmi les tricots 3D, on peut par exemple citer les produits commercialisés par la société Louis Vuidon.

La couche supplémentaire pourra contenir éventuellement des agents actifs qui contribuent à l'amélioration de la cicatrisation de l'ulcère de jambe ou qui permettent de diminuer la douleur ou l'oedème, ou encore des agents antibactériens. Selon une variante de réalisation, on pourra introduire dans la couche supplémentaire des fibres antibactériennes, par exemple des fibres argent, ou imprégner celle-ci avec un antibactérien par exemple du triclosan.

La structure aérée et la présence de boucles confèrent à la bande de l'invention d'excellentes propriétés d'amortissement et de conformabilité. Elle est également très discrète sous un pantalon grâce à sa faible épaisseur - de l'ordre de 2 mm par exemple. Grâce à cette faible épaisseur, le pied bandé peut aussi être facilement introduit dans une chaussure. Ces avantages augmentent l'acceptabilité du traitement par le patient.

De même, afin de garder ces avantages on préférera utiliser comme couche supplémentaire une ouate qui présente une épaisseur comprise entre 2 et 3 mm.

Selon un mode de réalisation préféré, afin de favoriser une pose précise par le personnel soignant, la bande de contention sera munie d'un moyen d'étalonnage. Ce moyen d'étalonnage peut être visuel, comme par exemple un ensemble de pictogrammes, régulièrement espacés, imprimés sur la bande ou réalisés au moyen d'un système d'étalonnage. Des informations sur les allongements à la pose recommandés peuvent être fournies avec le moyen d'étalonnage. L'étalonnage peut être réalisé par le personnel soignant sous la forme d'un pochoir. Ce type de pochoir ou les explications nécessaires pour le fabriquer peuvent être incorporés dans le conditionnement de la bande. Un kit comprenant plusieurs bandes de constitutions différentes, de largeurs différentes, de longueurs différentes et/ou dotées d'étalonnages différents pour appliquer des pressions spécifiques pourra être utilisé.

Lorsque le kit est indiqué pour le traitement des ulcères de jambes, il pourra en outre comprendre un ou plusieurs pansements destinés à être posés sur la plaie avant la pose de la bande.

Outre l'utilisation pour le traitement et la prévention des pathologies d'origine veineuse, en particulier les ulcères de jambes, ou le traitement des lymphoedèmes, en particulier les lymphoedèmes de la jambe pour lesquels il faut appliquer des pressions très élevées, les produits selon l'invention pourront être utilisés dans toute application où la conservation d'une pression appliquée est importante.

On peut ainsi citer le traitement et la prévention des pathologies traumatiques, des lésions articulaires, tendineuses, osseuses ou musculaires.

Accessoirement, selon l'objectif recherché, ces bandes pourront être utilisées, si nécessaire, dans un système de contention multicouche, par exemple bicouches, pour optimiser les propriétés de ce dernier ou éviter l'utilisation de bandes autoadhérentes à base de latex.

La bande de contention de l'invention permet d'obtenir des pressions de travail moyennes, voire même élevées, qui se maintiennent au cours du temps. Jusqu'à présent il était impossible de maintenir des pressions de travail élevées stables au cours du temps avec les bandes à allongement court.

Les performances des bandes de contention de l'invention peuvent être évaluées en termes de pressions de travail et de repos appliquées et de différentiel de pression, au cours du temps, en utilisant la méthode et l'appareil de test in vitro décrit dans la demande de brevet WO 2007/113430 page 17 ligne 26 à page 19 ligne 18.

Selon cette méthode la bande est posée autour d'un cylindre avec un recouvrement total de 100%, puis on fait varier la circonférence du cylindre à une vitesse imposée de façon continue entre une position dite de repos (diamètre le plus faible) et une position dite de travail (diamètre le plus grand) pour mimer la contraction musculaire. Des capteurs de pression mesurent au cours du temps les valeurs des pressions de repos et des pressions de travail.

L'écart de temps entre les mesures de pression de travail et de pression de repos est de 5 secondes et la fréquence des mesures de ces deux paramètres successifs est de 0,2Hz.

Pour tester les bandes de contention selon l'invention on peut déterminer l'allongement à la pose de la bande en fonction de la pression de travail recherchée, par exemple à l'aide de la courbe traction rupture telle que définie dans la norme EN 9073-3.

Pour poser la bande de façon appropriée, on peut étalonner les bandes à l'aide d'un pochoir comme décrit dans la demande de brevet WO 2007/113340 page 13 ligne 18 à page 14 ligne 6. Si nécessaire on peut affiner la valeur du pourcentage d'allongement à la pose par quelques tests successifs.

La valeur «Pression Max à T0» correspond à la première pression de travail enregistrée immédiatement après la pose et «Delta à T0»correspond au différentiel de pression entre la première pression de travail et la première pression de repos enregistrées immédiatement après la pose. Les valeurs «Pression Max à T24» et «Delta à T24» correspondent aux mesures enregistrées 24 heures après la pose. On calcule la différence de chacune de ces deux valeurs entre T0 et T24 heures «Pression Max (T0 - T24h)» et «Delta (T0 - T24h)».

On calcule également la perte de pression de travail à 24 heures «perte de Pression Max T24» par rapport à la pression de travail à la pose en faisant le rapport de la variation «Pression Max (T0 - T24h)» et de la «Pression Max à T0».

Les bandes de contention selon l'invention présentent avantageusement une pression de travail à 24 heures («Pression Max à T24») qui varie de 10 à 100 mm de mercure, par exemple de 15 à 25 mm de mercure ou de 25 à 85 mm de mercure.

En outre, les bandes de contention de l'invention présentent une excellente conservation de la pression appliquée à la pose au bout de 24 heures. La chute importante de cette valeur est généralement de l'ordre de 25 à 40 % au bout de 24 heures pour les bandes de contention à allongements courts de l'art antérieur, et de l'ordre de 20 à 25% pour les systèmes bicouches de l'art antérieur. Ainsi, la valeur «Pression Max (T0 - T24h)» des bandes de contention de la présente invention est avantageusement inférieure à 20%, par exemple comprise entre 10 et 15 %, de préférence inférieure à 10 %, voire même inférieure à 5%.

Des bandes de contention de l'invention telles que la pression à 24 heures («Pression Max à T24») est supérieure à 60 mm de mercure, et de préférence comprise entre 70 et 100 mm de mercure, sont utiles pour le traitement des lymphoedèmes, en particulier de la jambe.

La valeur des différentiels de pression à 24 heures («Delta à T24») des bandes de l'invention est avantageusement entre 10 et 45 mm de mercure, par exemple entre 30 et 35 mm de mercure.

Les bandes de l'invention présente de façon tout à fait inattendue une différence entre le Delta après la pose et le Delta à 24 heures («Delta (T0 - T24h)») négative. Ce résultat est d'autant plus remarquable qu'il est obtenu par une baisse de la pression de travail « Pression Max » moins rapide que celle de la pression de repos. Les bandes de contention selon l'invention sont donc les premières à améliorer leur efficacité au cours du temps.

Les bandes de l'invention permettent de traiter les ulcères mixtes, artériels et veineux en appliquant des pressions de travail faibles de l'ordre de 30 à 35 mm de mercure et en maintenant un différentiel de pression allant de 5 à 20 mm de mercure.

La bande de contention de l'invention est avantageusement telle qu'à une pression de travail «Pression Max à T0» équivalente à celle d'un autre système de contention de l'art antérieur, la perte de pression à T24 est diminuée, ce qui permet de changer la bande moins souvent. La bande de contention de l'invention présente les avantages des bandes à allongement court de l'art antérieur (fort différentiel de pression) et des bandes à allongement long de l'art antérieur (faible baisse de pression) sans leurs inconvénients. Elle est sous la forme d'une bande unique et non de plusieurs bandes.

La bande de contention de l'invention peut avantageusement combiner un différentiel de pression excellent («Delta à T24») typiquement compris entre 10 et 30 mm Hg, par exemple de l'ordre de 20 mm Hg, et une pression de travail faible («Pression Max à T0» et/ou («Pression Max à T24»)) comprise entre 30 et 40 mm Hg. Cette bande est donc facilement supportée et acceptée par les patients dans le traitement des ulcères de jambes classiques. De plus la présence de la ouate permet d'augmenter, si nécessaire, l'effet amortisseur de la bande et sa capacité d'absorption.

Les propriétés mécaniques de la bande de l'invention permettent également de l'utiliser pour plusieurs pathologies différentes, en faisant simplement varier son allongement.

On donnera maintenant divers exemples de bandes conformes à la présente invention.

### Exemples: Bandes de contention

Différents matériaux ont été utilisés pour fabriquer des bandes.

### 1. Matériaux utilisés

### -a) Non tissés

Les exemples utilisent deux non tissés différents, à base de fibres bicomposants asymétriques frisées, fabriqués selon l'enseignement de la demande de brevet WO 2008/015972. Ils portent respectivement les références SJJ 142 pour le non tissé A, et SJJ 146 pour le non tissé B chez la société Kuraray.

Ces deux non tissés sont réalisés à partir de la fibre, de type côte à côte, à base de copolymères polyesters de la société Kuraray dont la référence est PN -780.

Ces deux non tissés présentent les propriétés et les caractéristiques suivantes :

| | Non tissé A | Non tissé B |
|---|---|---|
| - Grammage (norme EN 9073-1) | 96 g/m² | 134 g/m² |
| - Epaisseur (norme EN 9073-2) | 1,13 mm | 1,14 mm |
| - Elasticité (norme EN 14704-1) | 86 % | 87 % |
| - Elongation longitudinale (norme EN 9073-3) | 117 % | 104 % |
| - Elongation transversale (norme EN 9073-3) | 111 % | 65 % |
| - Autoadhérence* | 0,03 N/cm | 0,03 N/cm |
| - Nombre de fibres frisées à la surface du non tissé** | 19 /cm² | 27 /cm² |

| | | |
|---|---|---|
| * mesurée selon la méthode décrite précédemment ** mesuré selon la méthode décrite précédemment | | |

### - b) Couche supplémentaire

Les matériaux utilisés pour la couche supplémentaire sont des produits commerciaux dont les dénominations ou références sont les suivantes et qui sont indiqués dans le tableau 1 en abrégé.
- mousses polyuréthane hydrophiles commercialisées sous la référence MCF.03 par la société AMS d'épaisseur 4,5 mm (en abrégé mousse 4,5 mm) et d'épaisseur 2,5 mm (en abrégé mousse 2,5 mm)
- ouate Ksoft® commercialisée par la société URGO Limited (en abrégé ouate)
- mousse hydrophobe commercialisée par la société ALVEO sous la dénomination ALVEOLIT® TEE.1000.8 (en abrégé mousse ALVEO)
- mousse polyuréthane commercialisée par la société SCAPA sous la dénomination Médifix® 4005 (en abrégé mousse SCAPA)
- tricot 3D commercialisé par la société Louis Vuidon sous la référence 9315 (en abrégé tricot 3D)
- film polyuréthane commercialisé par la société Leygatech sous la référence PU 55 IMPER 01 de 55 micromètres d'épaisseur en abrégé (film PU)

### 2. Assemblage

Différentes techniques d'assemblage ont été utilisées pour fabriquer les bandes: l'aiguilletage, l'application d'un adhésif par points ou l'assemblage par points par ultrasons.

### a) Conditions d'assemblage par aiguilletage

Les essais de complexage par aiguilletage ont été réalisés sur une machine à aiguilletage FEHRER en utilisant une planche à 2500 aiguilles par mètre linéaire.

Les essais ont été réalisés en deux passages sur la machine à aiguilleter, sauf pour l'exemple 6 qui ne comprend pas de couche supplémentaire.

Trois types d'aiguilletage différents ont été mis en oeuvre pour réaliser les bandes de contention de l'invention.

### Aiguilletage 1

Les conditions de réalisation sur la ligne d'aiguilletage 1 sont les suivantes:
- Vitesse sortie sur la ligne d'aiguilletage : 1 mètre/minute
- Pénétration des aiguilles : 10 mm
- Densité d'aiguilletage : 50 coups/cm²

L'association de la mousse et du non tissé avant aiguilletage 1 est mise en oeuvre sans précontrainte du non tissé.

### Aiguilletage 2

Les conditions de réalisation sur la ligne d'aiguilletage 2 sont les suivantes:
- Vitesse sortie sur la ligne d'aiguilletage : 1 mètre/minute
- Pénétration des aiguilles : 13 mm
- Densité d'aiguilletage : 20 coups/cm² avec le non tissé A et 40 coups/cm² avec le non tissé B

L'association de la ouate et du non tissé B ou du non tissé A avant aiguilletage 2 a été mise en oeuvre sans précontrainte du non tissé.

### Aiguilletage 3

Les conditions de réalisation sur la ligne d'aiguilletage 3 étaient les suivantes:
- Vitesse ligne = 1 m/mm
- Pénétration des aiguilles : 13 sauf pour l'exemple 10 où elle est de 18 mm
- Densité d'aiguilletage : 50 coups/cm²

L'association de la couche supplémentaire et du non tissé ou du non tissé avec lui-même avant aiguilletage 3 a été mise en oeuvre sans précontrainte du non tissé.

### b) Conditions d'assemblage par application d'adhésif à chaud par points avec un cylindre gravé

On a utilisé un assemblage avec un adhésif, lorsque la température d'application de l'adhésif nécessaire à sa dépose est compatible avec la résistance thermique du non tissé. Lorsque la bande comprend une couche supplémentaire, la surface de la couche supplémentaire doit être suffisamment régulière pour que l'adhésif puisse être réparti de façon régulière.

Le produit a été réalisé sur une machine CAVIMELT de contrecollage par cylindre gravé (partie gauche).
- Cylindre utilisé = cylindre N ° 6 Gravure Netz 1
- Conditions d'essais de la machine :
   Vitesse de marche = 2 m/min
   Fente de laminage = 0,3 mm
   Pression cylindre de laminage = 3 bars
   Pression du contre cylindre = 2,5 bars
   Température de chauffe = 188°C
   Température de la colle = 180°C

On a encollé la couche supplémentaire de mousse, puis on est venu contrecoller le non tissé. L'adhésif hot melt utilisé était une colle polyester de dénomination commerciale GRILTEX D 2116 E® de la société EMS.

### c) Conditions d'assemblage par ultrasons

On réalise un assemblage par points, qui correspondent au nombre de picots sur la surface du cylindre face à la sonotrode qui servent à faire la liaison ponctuelle entre les 2 non tissés.

Cet assemblage est réalisé sur un appareillage classique de la société HERRMANN ULTRASCHALLTECHNIK.

Les paramètres étaient les suivants :
- Cylindre de diamètre 190 mm dont la référence est H 058
- Sonotrode plane en titane non revêtue de largueur 161 mm
- Fréquence des ultrasons : 20 kHz
- Amplitude 100 %
- Vitesse de passage de l'ordre de 5 m/min.

L'ensemble des bandes réalisées et les techniques d'assemblage sont rassemblées dans le tableau 1.

**Tableau 1**

| Exemple | Non tissés utilisés | Couche supplémentaire | assemblage |
|---|---|---|---|
| 1 | B / B | Mousse 4,5mm | Aiguilletage 1 |
| 2 | B / B | Mousse 4,5mm | Adhésif |
| 3 | B / B | Ouate | Aiguilletage 2 |
| 4 | A / B | Ouate | Aiguilletage 2 |
| 5 | A / A | Ouate | Aiguilletage 2 |
| 6 | B / B | Sans | Aiguilletage 3 |
| 7 | B / B | Mousse 2,5mm | Aiguilletage 3 |
| 8 | B / B | Mousse SCAPA | Aiguilletage 3 |
| 9 | B / B | Mousse alvéo | Aiguilletage 3 |
| 10 | B / B | Tricot 3D | Aiguilletage 3 |
| 11 | B / B | Film PU | Aiguilletage 3 |
| 12 | B / B | Sans | Ultrasons |

### 3. Performance des bandes de contention

Les performances des bandes de contention des Exemples 1 à 12 ont été évaluées en termes de pressions de travail et de repos et de différentiel de pression, au cours du temps.

On a utilisé la méthode et l'appareil de test in vitro décrit dans la demande de brevet WO 2007/113430 page 17 ligne 26 à page 19 ligne 18. Selon cette méthode la bande est posée autour d'un cylindre avec un recouvrement total de 100%, puis on fait varier la circonférence du cylindre à une vitesse imposée de façon continue entre une position dite de repos (diamètre le plus faible) et une position dite de travail (diamètre le plus grand) pour mimer la contraction musculaire. Des capteurs de pression mesurent au cours du temps les valeurs des pressions de repos et des pressions de travail.

L'écart de temps entre les mesures de pression de travail et de pression de repos est de 5 secondes et la fréquence des mesures de ces deux paramètres successifs est de 0,2Hz.

Pour tester les bandes de contention selon l'invention on a déterminé l'allongement à la pose de la bande en fonction de la pression de travail recherchée, par exemple à l'aide de la courbe traction rupture telle que définie dans la norme EN 9073-3. D'après la loi de Laplace, l'allongement à effectuer correspond à la pression recherchée.

Pour poser la bande de façon appropriée, on a étalonné les bandes à l'aide d'un pochoir comme décrit dans la demande de brevet WO 2007/113340 page 13 ligne 18 à page 14 ligne 6. Si nécessaire on a affiné la valeur du pourcentage d'allongement à la pose par quelques tests successifs.

Chacune des bandes a été posée à un allongement donné, exprimé en pourcentage, qui est indiqué dans le tableau 2.

La valeur «Pression Max à T0» correspond à la première pression de travail enregistrée immédiatement après la pose et «Delta à T0»correspond au différentiel de pression entre la première pression de travail et la première pression de repos enregistrées immédiatement après la pose. Les valeurs «Pression Max à T24» et «Delta à T24» correspondent aux mesures enregistrées 24 heures après la pose. Puis on a calculé la différence de chacune de ces deux valeurs entre T0 et T24 heures «Pression Max (T0 - T24h)» et «Delta (T0 - T24h)».

On a aussi calculé la perte de pression de travail à 24 heures «perte de Pression Max T24» par rapport à la pression de travail à la pose en faisant le rapport de la variation «Pression Max (T0 - T24h)» et de la «Pression Max à T0».

On a comparé les performances des bandes selon l'invention aux systèmes de contention bicouches commercialisés par la société URGO Limited sous les dénominations K2® et K2 Lite®. Les bandes de ces produits commerciaux étaient déjà étalonnées.

L'ensemble des résultats a été reporté dans le tableau 2.

**Tableau 2**

| Bande utilisée | Allongement à la pose | Pression Max à T0 (mm Hg) | Delta à T0 (mm Hg) | Pression Max à T24 (mm Hg) | Delta à T24 (mm Hg) | Delta (T0-T24) (mm Hg) | Pression Max (T0-T24) (mm Hg) | Perte de pression Max T24 |
|---|---|---|---|---|---|---|---|---|
| K2 | 55% + 50% | 44 | 19 | 35 | 17 | +2 | 9 | 20,4% |
| K2 Lite | 50% + 50% | 33 | 10 | 25 | 8 | +2 | 8 | 24,2% |
| Exemple 1 | 30% | 93 | 23 | 81 | 27 | -4 | 11 | 11,8% |
| Exemple 2 | 30% | 67 | 21 | 58 | 22 | -1 | 9 | 13,4% |
| Exemple 3 | 20% | 77 | 30 | 71 | 34 | -4 | 6 | 7,8% |
| Exemple 4 | 20% | 39 | 17 | 37 | 20 | -3 | 2 | 5,1% |
| Exemple 5 | 20% | 39 | 17 | 33 | 19 | -2 | 6 | 15,3% |
| Exemple 6 | 30% | 54 | 17 | 47 | 20 | -3 | 7 | 13% |
| Exemple 6 | 20% | 48 | 15 | 46 | 18 | -3 | 2 | 4,2% |
| Exemple 6 | 15% | 33 | 12 | 29 | 14 | -2 | 4 | 12,1% |
| Exemple 12 | 20% | 52 | 13 | 50 | 15 | -2 | 2 | 3,8% |
| Exemple 7 | 30% | 66 | 17 | 59 | 21 | -4 | 7 | 10,6% |
| Exemple 8 | 30% | 82 | 24 | 68 | 28 | -4 | 14 | 17% |
| Exemple 9 | 10% | 76 | 29 | 70 | 39 | -10 | 6 | 7,9% |
| Exemple 10 | 30% | 71 | 22 | 61 | 27 | -5 | 7 | 14% |
| Exemple 11 | 10% | 70 | 25 | 63 | 30 | -5 | 7 | 10% |

### Interprétation des résultats

L'analyse des résultats du tableau 2 démontre les performances des bandes de contention selon l'invention.

De façon générale ces résultats montrent que l'on peut traiter toutes les pathologies précédemment décrites avec une seule bande de contention autoadhérente sans latex ou adhésif car on a, selon les produits ou leur allongement à la pose, une plage de valeur de la pression de travail à 24 heures qui varie de 29 à 81 mm de mercure.

On constate aussi que toutes ces bandes de contention présentent une excellente conservation de la pression appliquée à la pose au bout de 24 heures.

La chute importante qui est généralement constaté pour les bandes à allongements courts, de l'ordre de 30 à 40 % au bout de 24 heures, et de l'ordre de 20 à 25% pour les systèmes bicouches qui sont les plus performants, est ici beaucoup plus faible. Elle est toujours inférieure à 20% et le plus souvent comprise entre 10 et 15 %, voire même inférieure à 10 % pour les exemples 3, 4, 6 (posé à 20%), 9 et 12.

Pouvoir appliquer une pression élevée et la conserver au cours du temps est un paramètre très important pour le traitement des lymphoedèmes, en particulier les lymphoedèmes de la jambe, pour lesquels on souhaite à 24 heures une pression supérieure à 60 mm de mercure, et de préférence comprise entre 70 et 100 mm de mercure.

Les exemples 1, 3, 8, 9, 10 et 11 sont particulièrement bien adaptés au traitement de cette pathologie.

De même on constate que la valeur des différentiels de pression à 24 heures varie entre 14 et 39 mm de mercure ce qui permet de s'adapter à toutes les catégories d'ulcères de jambes indiquées précédemment.

On constate aussi que le différentiel de pression entre pression de travail et pression de repos ne diminue pas au cours du temps mais au contraire, de façon inattendue, augmente. Ainsi pour toutes les bandes de l'invention, la différence entre le Delta après la pose et le Delta à 24 heures est négative.

Ce résultat est d'autant plus remarquable qu'il est obtenu par une baisse de la pression de travail «Pression max» (qui garantit l'efficacité du système) moins rapide que celle de la pression de repos.

Les bandes de contention selon l'invention sont donc les premières à améliorer leur efficacité au cours du temps.

On peut aussi constater que ces résultats sont obtenus avec des produits à base de matériaux différents ou assemblés selon des technologies différentes.

Ainsi si l'on vise une bande qui présente un différentiel de pression à 24 heures de l'ordre de 15 à 25 mm de mercure, les bandes de contention des exemples 2, 4, 5, 6, 7 et 12 peuvent remplir ce cahier des charges.

En particulier l'exemple 6 qui correspond à l'aiguilletage de deux non tissés B est particulièrement intéressant car, posé à 20 %, il donne des résultats équivalents voire même meilleurs en termes de delta et de pression à 24 heures au produit K2® en utilisant une seule bande. Il présente aussi une des chutes de pression de travail à 24 heures les plus faibles à 4,2%.

De même les exemples 4 et 5 constitués respectivement des non tissés A et B avec une ouate, et de deux non tissés A avec une ouate donnent, posés à 20%, à 24 heures, des différentiels de pression excellents de 20 et 19 mm de mercure, tout en appliquant immédiatement après la pose des pressions de travail faibles de l'ordre de 39 mm de mercure. Ces bandes seront donc facilement supportées et acceptées par les patients dans le traitement des ulcères de jambes classiques. De plus la présence de la ouate permet d'augmenter, si nécessaire, l'effet amortisseur de la bande et sa capacité d'absorption.

L'exemple 6 est aussi intéressant car on constate qu'avec une seule bande posée à des allongements différents on peut couvrir plusieurs pathologies.

Pour pouvoir traiter les ulcères mixtes, artériels et veineux on souhaite - à cause de la composante artérielle - des pressions de travail faibles (de l'ordre de 30 à 35 mm de mercure) tout en conservant un différentiel de pression élevé. Pour obtenir ce résultat, il a été nécessaire de développer un système de contention bicouches spécifique le K2 Lite®.

On constate que la bande de l'exemple 6 posée à 15 % d'allongement permet d'obtenir des valeurs de pression dans la fourchette recherchée et du même ordre que le K2 Lite® tout en conservant un différentiel de pression plus élevé que celui du K2 Lite®.

On constate donc qu'avec une seule bande, en jouant sur son allongement à la pose, on peut obtenir à la fois un système équivalent ou supérieur au système K2 mais aussi un système utilisable pour la pathologie des ulcères mixtes et que ce système est plus efficace que le système K2 Lite®.

Tous les résultats obtenus démontrent que l'on a enfin réalisé une bande de contention qui présente les avantages des bandes à allongement court (fort différentiel de pression) et des bandes à allongement long (faible baisse de pression) sans leurs inconvénients avec une seule bande. Cette bande est plus performante que les meilleurs systèmes de contention bicouches connus.

## Revendications

1. Bande de contention comprenant deux non tissés de fibres frisées obtenues à partir de fibres courtes conjuguées, dans laquelle
- les non tissés sont assemblés l'un à l'autre et ont indépendamment l'un de l'autre un grammage compris entre 70 g/m² et 300 g/m²,
- lesdites fibres sont frisées de façon uniforme dans le sens de l'épaisseur des non tissés, et présentent un rayon de courbure moyen compris entre 10 et 200 micromètres, et
- le nombre de fibres frisées à la surface de chacun des non-tissés est supérieur à 10 fibres frisées/cm².

2. Bande de contention selon la revendication 1, **caractérisée en ce que** les fibres courtes conjuguées sont bicomposants, les deux composants les constituant étant des polymères qui présentent un point de ramollissement supérieur ou égal à 100°C, lesdits polymères étant choisis parmi les polymères polypropyléniques, les polymères polyesters et/ou les polymères polyamides, et de préférence sont deux polymères polyesters aromatiques différents.

3. Bande de contention selon la revendication 2, **caractérisée en ce que** les fibres bicomposants ont une structure de type côte à côte et sont constituées d'un premier polymère qui est un polyéthylène téréphtalate, et d'un second polymère qui est un copolymère d'un alkylène arylate avec l'acide isophtalique et/ou du diéthylène glycol.

4. Bande de contention selon la revendication 1, **caractérisée en ce que** le titre moyen des fibres courtes conjuguées est compris entre 1 et 5 dtex, de préférence entre 1,5 et 3 dtex, et la longueur moyenne des fibres courtes conjuguées est comprise entre 10 à 100 mm, et de préférence entre 40 et 60 mm.

5. Bande de contention selon la revendication 1, **caractérisée en ce que** les fibres frisées présentent un rayon de courbure moyen compris entre 50 et 160 microns, et de préférence entre 70 et 130 microns.

6. Bande de contention selon la revendication 1, **caractérisée en ce que** chaque non tissé a indépendamment l'un de l'autre un grammage compris entre 80 et 200 g/m² et de préférence entre 90 et 150 g/m².

7. Bande de contention selon la revendication 1, **caractérisée en ce que** le nombre de fibres frisées à la surface de chacun des non tissés est compris entre 10 et 50 fibres frisées / cm² et de préférence entre 10 et 35 fibres frisées / cm².

8. Bande de contention selon la revendication 1, **caractérisée en ce que** chaque non tissé présente dans une section prise dans le sens de l'épaisseur, un ratio d'incurvation de fibre supérieur ou égal à 1,3 dans chacun des domaines délimités par un partage en trois parts égales dans le sens de l'épaisseur, et le rapport de la valeur minimale à la valeur maximale du ratio d'incurvation de fibre dans les différents domaines est supérieur à 75 %.

9. Bande de contention selon la revendication 1, **caractérisée en ce qu'**elle comprend une couche supplémentaire choisie parmi les matériaux textiles, les matériaux alvéolaires, les films ou leurs combinaisons.

10. Bande de contention selon la revendication 9, **caractérisée en ce que** le matériau textile est un non tissé inélastique, de préférence absorbant, qui présente une épaisseur supérieure à 1,8 mm, de préférence comprise entre 1,8 et 4 mm, et en particulier comprise entre 2 et 3 mm.

11. Bande de contention selon la revendication 9, **caractérisée en ce que** la couche supplémentaire est une mousse de polyuréthane hydrophile ou une ouate.

12. Bande de contention selon la revendication 1, **caractérisée en ce que** les non tissés sont assemblés par aiguilletage, avec un adhésif ou par ultra-sons.

13. Bande de contention selon la revendication 1, **caractérisée en ce qu'**elle comprend deux non tissés, de préférence identiques, constitués de fibres bicomposants côte à côte à base de polymères polyesters aromatiques, chaque non tissé ayant un grammage compris entre 90 et 150 g/m² et le nombre de fibres frisées à la surface de chaque non tissé est compris entre 10 et 35 fibres frisées/cm².

14. Bande de contention selon la revendication 9, **caractérisée** en ce les deux non tissés sont aiguilletés avec une couche supplémentaire qui est une ouate présentant une épaisseur comprise entre 2 et 3 mm.

15. Bande de contention selon l'une de revendications précédentes, pour son utilisation dans le traitement des ulcères de jambes et des lymphoedèmes.

## Patentansprüche

1. Stützbandage, umfassend zwei Vliese aus gekräuselten Fasern, die aus kurzen konjugierten Fasern erhalten werden,
- wobei die Vliese miteinander verbunden sind und unabhängig voneinander ein Flächengewicht zwischen 70 g/m² und 300 g/m² haben,
- wobei die gekräuselten Fasern einheitlich in die Richtung der Dicke der Vliese gekräuselt sind und einen durchschnittlichen Krümmungsradius zwischen 10 und 200 Mikrometer aufweisen, und
- wobei die Anzahl von gekräuselten Fasern an der Oberfläche des Vlieses größer als 10 gekräuselte Fasern/cm² ist.

2. Stützbandage gemäß Anspruch 1, **dadurch gekennzeichnet, dass** die konjugierten kurzen Fasern Zweikomponentenfasern sind, wobei die zwei Komponenten, aus denen sie bestehen, Polymere sind, die einen Erweichungspunkt größer oder gleich 100°C aufweisen, wobei die Polymere unter den Polypropylen-Polymeren, den Polyester-Polymeren und/oder den Polyamid-Polymeren ausgewählt und vorzugsweise zwei unterschiedliche aromatische Polyester-Polymere sind.

3. Stützbandage gemäß Anspruch 2, **dadurch gekennzeichnet, dass** die Zweikomponentenfasern eine Seite-an-Seite-Struktur haben und von einem ersten Polymer, das ein Polyethylen-Terephthalat ist, und einem zweiten Polymer, das ein Compolymer eines Alkylenarylats mit der Isophthalsäure und/oder Diethylenglykol ist, gebildet sind.

4. Stützbandage gemäß Anspruch 1, **dadurch gekennzeichnet, dass** der durchschnittliche Titer der konjugierten kurzen Fasern zwischen 1 und 5 dtex, vorzugsweise zwischen 1,5 und 3 dtex, beträgt, und die durchschnittliche Länge der konjugierten kurzen Fasern zwischen 10 und 100 mm und vorzugsweise zwischen 40 und 60 mm beträgt.

5. Stützbandage gemäß Anspruch 1, **dadurch gekennzeichnet, dass** die gekräuselten Fasern einen durchschnittlichen Krümmungsradius zwischen 50 und 160 Mikrometer und vorzugsweise zwischen 70 und 130 Mikrometer aufweisen.

6. Stützbandage gemäß Anspruch 1, **dadurch gekennzeichnet, dass** jedes Vlies unabhängig voneinander ein Flächengewicht zwischen 80 und 200 g/m² und vorzugsweise zwischen 90 und 150 g/m² aufweist.

7. Stützbandage gemäß Anspruch 1, **dadurch gekennzeichnet, dass** die Anzahl von gekräuselten Fasern an der Oberfläche jedes der Vliese zwischen 10 und 50 gekräuselte Fasern/cm² und vorzugsweise zwischen 10 und 35 gekräuselte Fasern/cm² beträgt.

8. Stützbandage gemäß Anspruch 1, **dadurch gekennzeichnet, dass** jedes Vlies in einem in Richtung der Dicke genommenen Querschnitt ein Faserkrümmungsverhältnis größer oder gleich 1,3 in jedem der Bereiche aufweist, die durch eine Teilung in drei gleiche Teile in Richtung der Dicke begrenzt sind, und dass das Verhältnis des Mindestwerts zum Höchstwert des Faserkrümmungsverhältnisses in den verschiedenen Bereichen größer als 75 % ist.

9. Stützbandage gemäß Anspruch 1, **dadurch gekennzeichnet, dass** sie eine zusätzliche Schicht umfasst, die unter den Textilmaterialien, den Schaumstoffen, den Folien oder ihren Kombinationen ausgewählt ist.

10. Stützbandage gemäß Anspruch 9, **dadurch gekennzeichnet, dass** das Textilmaterial ein nicht elastisches, vorzugsweise absorbierendes, Vlies ist, das eine Dicke von mehr als 1,8 mm, vorzugsweise zwischen 1,8 und 4 mm und insbesondere zwischen 2 und 3 mm, aufweist.

11. Stützbandage gemäß Anspruch 9, **dadurch gekennzeichnet, dass** das die zusätzliche Schicht ein hydrophiler Polyurethanschaum oder eine Watte ist.

12. Stützbandage gemäß Anspruch 1, **dadurch gekennzeichnet, dass** die Vliese durch Nadeln, mittels eines Haftmittels oder durch Ultraschall verbunden werden.

13. Stützbandage gemäß Anspruch 1, **dadurch gekennzeichnet, dass** sie zwei Vliese umfasst, die vorzugsweise identisch sind, die aus Seite-an-Seite-Zweikomponentenfasern auf Basis von aromatischen Polyester-Polymeren gebildet sind, wobei jedes Vlies ein Flächengewicht zwischen 90 und 150 g/m² hat, und die Anzahl von gekräuselten Fasern an der Oberfläche jedes Vlieses zwischen 10 und 35 gekräuselte Fasern/cm² beträgt.

14. Stützbandage gemäß Anspruch 13, **dadurch gekennzeichnet, dass** die beiden Vliese mit einer zusätzlichen Schicht genadelt sind, die eine Watte ist, die eine Dicke zwischen 2 und 3 mm aufweist.

15. Stützbandage gemäß einem der vorhergehenden Ansprüche zur Verwendung bei der Behandlung von Beingeschwüren oder Lymphödemen.

## Claims

1. A compression bandage comprising two nonwovens of crimped fibers obtained from short conjugate fibers, in which:
- the two nonwovens are assembled together and have, independently of one another, a grammage of between 70 g/m² and 300 g/m²,
- the crimped fibers are uniformly crimped in the thickness direction of the nonwovens, and exhibit a mean curvature radius of between 10 and 200 micrometers, and
- the number of crimped fibers at the surface of each of the nonwovens is greater than 10 crimped fibers/cm².

2. The compression bandage as claimed in claim 1, **characterized in that** the short conjugate fibers are bicomponent fibers that are made of two polymer components which exhibit a softening point greater than or equal to 100°C, and which are selected in the group consiting of polypropylene polymers, polyester polymers and polyamide polymers, and which are preferably two different aromatic polyester polymers.

3. The compression bandage as claimed in claim 2, **characterized in that** the bicomponent fibers have a side by side type structure and are composed of a first polymer which is a polyethylene terephthalate and of a second polymer which is a copolymer of an alkylene arylate with isophthalic acid and/or diethylene glycol.

4. The compression bandage as claimed in claim 1, **characterized in that** the short conjugate fibers have a mean count of between 1 and 5 dtex, preferably between 1.5 and 3 dtex, and a mean length of between 10 and 100 mm and preferably between 40 and 60 mm.

5. The compression bandage as claimed in claim 1, **characterized in that** the crimped fibers exhibit a mean curvature radius of between 50 and 160 microns and preferably between 70 and 130 microns.

6. The compression bandage as claimed in claim 1, **characterized in that** each nonwoven has, independently of one another, a grammage of between 80 and 200 g/m² and preferably between 90 and 150 g/m².

7. The compression bandage as claimed in claim 1, **characterized in that** the number of crimped fibers at the surface of each of the nonwovens is between 10 and 50 crimped fibers/cm² and preferably between 10 and 35 crimped fibers/cm².

8. The compression bandage as claimed in claim 1, **characterized in that** each nonwoven exhibits, in a cross-section taken parallel to the thickness direction thereof,
- a fiber incurvation ratio greater than or equal to 1.3, and
- a ratio between the minimum value of the fiber incurvation ratio and the maximum value of the fiber incurvation ratio greater than 75%,
wherein said ratio and said fiber invurvation ratio are measured in three parts of the non woven, each part corresponding to one third thereof in a cross-section taken perpendicular to the thickness direction thereof.

9. The compression bandage as claimed in claim 1, **characterized in that** it comprises a supplementary layer selected in the group consistin of textile materials, cellular materials, films, and their combinations.

10. The compression bandage as claimed in claim 9, **characterized in that** the textile material is a nonelastic nonwoven, preferably an absorbent nonwoven, which exhibits a thickness greater than 1.8 mm, preferably between 1.8 and 4 mm and in particular between 2 and 3 mm.

11. The compression bandage as claimed in claim 9, **characterized in that** the supplementary layer is a hydrophilic polyurethane foam or a padding.

12. The compression bandage as claimed in claim 1, **characterized in that** the nonwovens are assembled by needling, with an adhesive or by ultrasound.

13. The compression bandage as claimed in claim 1, **characterized in that** it comprises two nonwovens, preferably identical, composed of side by side bicomponent fibers that are based on aromatic polyester polymers, each nonwoven having a grammage between 90 and 150 g/m² and the number of crimped fibers at the surface of each nonwoven being between 10 and 35 crimped fibers/cm².

14. The compression bandage as claimed in claim 9, **characterized in that** the two nonwovens are needled with a supplementary layer which is a padding exhibiting a thickness between 2 and 3 mm.

15. The compression bandage as claimed in one of the preceding claims, for the use thereof in the treatment of leg ulcers and lymphedema.
